# EUROPÄISCHE PATENTSCHRIFT

(11) **EP 2 990 002 B1**
(45) Veröffentlichungstag und Bekanntmachung des Hinweises auf die Patenterteilung: **15.08.2018**
(21) Anmeldenummer: 15183181.5
(22) Anmeldetag: 31.08.2015
(51) Int. Cl.: A61B 17/72, A61B 17/00

(54) **MARKNAGEL**
INTRAMEDULLARY NAIL
CLOU MEDULLAIRE

(30) Priorität: 01.09.2014 DE 102014112573
(43) Veröffentlichungstag der Anmeldung: 02.03.2016
(73) Patentinhaber: WITTENSTEIN SE, 97999 Igersheim (DE)
(72) Erfinder: Stauch, Roman, 97959 Assamstadt (DE)
(74) Vertreter: Zimmermann & Partner Patentanwälte mbB

(56) Entgegenhaltungen:
- EP-A2- 2 570 092
- WO-A1-95/22292
- WO-A1-2009/152270
- WO-A2-2011/116158
- US-A- 5 074 882
- US-A1- 2006 293 683
- US-B2- 7 063 706

## Beschreibung

### Gebiet der Erfindung

Die Erfindung betrifft einen Marknagel für eine Distraktion und anschließender Verlängerung eines Röhrenknochens mit Segmenttransport.

### Stand der Technik

Aus dem Stand der Technik, wie der DE 10 2011 053 638 A1, sind Marknägel bekannt, welche eine Distraktion von langen Röhrenknochen mit Segmenttransport ermöglichen. Zwei Knochenfragmente, ein distales Endfragment und ein proximales Endfragment, werden relativ zueinander mittels eines Marknagels verschoben. Weiterhin existiert bei Anwendungen mit Segmenttransport ein Mittelfragment des Knochens, welches auch als Segment bezeichnet wird und ebenfalls Bestandteil des frakturierten Knochens ist. An einer Kontaktstelle zwischen einem der Knochenendfragmente und dem Mittelfragment soll Knochen nachwachsen. Dies wird erreicht, indem die Vorschubgeschwindigkeit des Mittelfragments zur Distraktion ausreichend klein gewählt wird.

Solche Verschiebungen ermöglichen eine Behandlung von großen Knochendefekten von mehr als beispielsweise 3 cm, wie sie in Folge von Krankheiten oder Gewalteinwirkung entstehen können. Auch bei Operationen aufgrund von Knochentumoren können große Knochendefekte entstehen, welche je nach Schweregrad der Erkrankung mit einem Marknagel mit Segmentverschiebung behandelt werden können.

Aus der US 2006/0293683 A1 ist ein Marknagel bekannt, welcher zwischen zwei Endfragmenten eine doppelte Segmentverschiebung bewirken kann. Die US 5,074,882 A offenbart einen Marknagel ohne Segmenttransport. Die WO 2009/152270 A1 offenbart Claviculanägel, welche Verankerungselemente zum Verankern des Nagels im Knochen aufweisen.

Eine Herausforderung bei Marknägeln mit einem Transport eines Mittelfragments sind grundsätzlich die Fixierungen der Enden des Marknagels in dem proximalen Knochenfragment und dem distalen Knochenfragment. Besonders bei kurzen Fragmenten an den Enden des Knochens können aus dem Stand der Technik bekannte Marknägel schwierig zu verankern sein.

### Aufgabe der Erfindung

Aufgabe der Erfindung ist es, einen Marknagel anzugeben, wobei aus dem Stand der Technik bekannte Systeme oder Marknägel verbessert werden sollen, insbesondere sollen die Nachteile des Standes der Technik gelindert oder behoben werden. Wünschenswert sind Marknägel, welche einen möglichst geringen Operationsaufwand benötigen oder eine zuverlässige Fixierung auch in kurzen Knochenendfragmenten ermöglichen.

Die Aufgabe wird mit einem Marknagel nach dem Anspruch 1 gelöst.

Ein Aspekt der Erfindung betrifft einen Marknagel für eine Distraktion und anschließender Verlängerung eines Röhrenknochens mit Mittelfragmenttransport, wobei der Marknagel umfasst: einen sich in einer axialen Richtung des Marknagels erstreckenden zumindest teilweise hohlen Rumpf, ein Verriegelungsmittel zum Verriegeln des Rumpfes in einem ersten Endfragment des Röhrenknochens, ein erstes Innenteil, welches innerhalb des Rumpfes in axialer Richtung verschiebbar angeordnet ist und ein erstes Fixierungsmittel zum Fixieren eines Mittelfragments des Röhrenknochens umfasst, ein zweites Innenteil, welches innerhalb des Rumpfes in axialer Richtung verschiebbar angeordnet ist und ein zweites Fixierungsmittel zum Fixieren eines zweiten Endsegments des Röhrenknochens umfasst, und einen Antrieb zum axialen Verschieben des ersten Innenteils relativ zu dem zweiten Innenteil, wobei der Antrieb innerhalb des Rumpfes zwischen dem ersten Fixierungsmittel des ersten Innenteils und dem zweiten Fixierungsmittel des zweiten Innenteils angeordnet ist.

Beispielhafte Ausführungsformen von Marknägeln weisen einen sich in axialer Richtung des Marknagels erstreckenden hohlen Rumpf auf. Der hohle Rumpf ermöglicht die Aufnahme von verschiebbaren Innenteilen.

Bei typischen Ausführungsformen ist der Antrieb integral mit dem zweiten Innenteil ausgeführt. Bei weiteren typischen Ausführungsformen ist der Antrieb integral mit dem ersten Innenteil ausgeführt. Der Begriff "integral ausgeführt" bedeutet insbesondere, dass der Antrieb in den Innenteil integriert ist. Eine integrale Ausführung des Antriebs mit dem Innenteil kann verwendet werden, um den Antrieb in einem der Innenteile zu integrieren. Der Antrieb ist bei typischen Ausführungsformen schwimmend ausgeführt. Das bedeutet insbesondere, dass der Antrieb in axialer Richtung verschieblich ist. Typischerweise ist der Antrieb mit dem Innenteil verschieblich, in welches er integriert ist. Bei weiteren Ausführungsformen ist der Antrieb ein von den Innenteilen separates Bauteil. Beispielsweise liegt der Antrieb mit einer Seite ohne Befestigung oder mit einer Fixierung an einem der Innenteile an. Typischerweise ist auf einer Seite des Antriebs eine Spindel angeordnet, welche mit einem Innengewinde in einem der Innenteile interagiert. Auf diese Weise kann der Antrieb die beiden Innenteile voneinander wegbewegen. Bei einer an einem Innenteil fixierten oder bei einer integralen Ausführung kann der Antrieb auch verwendet werden, um die Innenteile aufeinander zuzubewegen. Bei Ausführungsformen ist ein zweiter Antrieb vorgesehen. Zwei Antriebe können verwendet werden, um die Innenteile unabhängiger voneinander an bestimmte Positionen zu verfahren.

Bei typischen Ausführungsformen ist der mindestens eine Antrieb zwischen den Fixierungsmitteln der Innenteile angeordnet. Auf diese Weise kann das zweite Innenteil verwendet werden, auch um mit kurzen Endfragmenten verbunden zu werden. Bei einer integralen Ausführung des Antriebs in einem der Innenteile ist der Antrieb auf der jeweils dem anderen Innenteil zugewandten Seite angeordnet.

Typischerweise sind in dem mittleren Bereich des Rumpfes ein erstes Paar axial ausgerichtete, gegenüberliegende Langlöcher angeordnet. Dies ermöglicht eine Durchführung von mindestens einem radial ausgerichteten Fixierungsbolzen oder einer Schraube zur Befestigung eines Mittelfragments. Das Mittelfragment kann auch als Transportsegment bezeichnet werden. Der Begriff "mittlerer Bereich" bezieht sich hierin auf einen Bereich, welcher nicht an einem Ende des Rumpfes angeordnet ist. Insbesondere bezieht sich "mittlerer Bereich" nicht nur auf die geometrische Mitte des Rumpfes. Vielmehr erstreckt sich der "mittlere Bereich" über einen größeren Bereich als lediglich die exakte geometrische Mitte. Bei typischen Ausführungsformen sind in dem zweiten Endbereich des Rumpfes ein zweites Paar axial ausgerichtete, gegenüberliegende Langlöcher angeordnet sind. Auf diese Weise lassen sich in radialer Richtung einer oder mehrere Fixierungsbolzen zum Fixieren eines zweiten Endfragmentes durch den Rumpf und durch das zweite Innenteil hindurchführen, um das zweite Endfragment mit dem zweiten Innenteil zu verbinden. Durch das Anordnen von Langlöchern wird ermöglicht, dass mit einem oder mehreren Fixierungsbolzen ein Knochenfragment, also beispielsweise ein Mittelfragment oder ein Endfragment fixiert werden können. Das Mittelfragment ist dabei üblicherweise ein Teil des Knochens, welcher zwischen einem ersten Endfragment und einem zweiten Endfragment angeordnet ist. Bei Ausführungsformen kann das erste Endfragment ein proximales Knochenfragment sein oder ein distales Knochenfragment. Dementsprechend ist das zweite Endfragment dann ein distales Knochenfragment oder im anderen Fall ein proximales Knochenfragment.

Beispielhafte Marknägel eignen sich insbesondere zur Behandlung von Frakturen oder anderen Schädigungen von langen Röhrenknochen, wobei andere Schädigungen beispielsweise Knochenverluste aufgrund von Tumoren, notwendigen Resektionen oder Gewalteinwirkung sein können. Knochen, welche mit typischen Marknägeln behandelt werden können, sind Oberschenkelknochen (Femur) und Schienenbein (Tibia), es können jedoch auch Oberarmknochen (Humerus), Elle (Ulna), Speiche (Radius) und Wadenbein (Fibula) behandelt werden. Typische hier beschriebene Marknägel sind besonders auch für kleinwüchsige oder minderjährige Patienten geeignet, da der Aufbau beispielhafter Marknägel der Erfindung eine vergleichsweise geringe Länge der Knochenfragmente zur Verriegelung benötigt.

Typische Marknägel der Erfindung weisen ein Verriegelungsmittel zum Verriegeln des Rumpfes in dem ersten Endfragment des Röhrenknochens auf. Auf diese Weise kann der Rumpf mit einem ersten Endfragment des Röhrenknochens in alle Richtungen und alle Drehrichtungen fixiert verbunden werden. Der Rumpf ist damit in allen Freiheitsgraden mit dem Knochenfragment fixiert verbunden. Typischerweise ist das Verriegelungsmittel in einem ersten Endbereich des Rumpfes angeordnet. Damit wird eine Fixierung eines Endfragments ermöglicht. Das Verriegelungsmittel des Rumpfes umfasst bei Ausführungsformen radial ausgerichtete Bohrungen, beispielsweise zumindest zwei, typischerweise zueinander um die Längsachse des Marknagels verdreht angeordnete, Durchgangs- oder Sacklochbohrungen. Das Verriegelungsmittel umfasst bei Ausführungsformen Bolzen oder Schrauben, welche in den Bohrungen aufgenommen werden können. Die Schrauben oder Bolzen ermöglichen eine Verankerung des Rumpfes in dem ersten Endfragment.

Bei typischen Ausführungsformen umfasst das Verriegelungsmittel Bohrungen oder Verriegelungsbolzen, welche ausschließlich radial ausgerichtet sind. Ausführungsformen weisen ausschließlich Bohrungen, welche einen Winkel, insbesondere einen Winkel von mindestens 30°, mindestens 50° oder mindestens 80° mit der Längsachse des Marknagels einschließen, auf. Radial ausgerichtete Bolzen bieten guten Halt im Knochen. Bei weiteren Ausführungsformen sind zusätzlich längs zum Marknagel ausgerichtete Bolzen oder Schrauben vorgesehen, um die Verriegelung zu stabilisieren.

Als Antrieb wird typischerweise ein elektrischer Motor mit Getriebe vorgesehen. Typische Getriebe sind Planetengetriebe, weitere Ausführungsformen umfassen Planetenrollengetriebe. Zur Energieversorgung und Steuerung des Antriebs ist typischerweise eine Kontrolleinheit vorgesehen, welche drahtlos von außerhalb eines Körpers, in welchen der Marknagel eingesetzt ist, über eine Antenne mit Energie oder Steuersignalen versorgt werden kann. Bei Ausführungsformen ist die Kontrolleinheit dazu in der Lage, erfasste Größen, wie beispielsweise eine zur Verschiebung erforderliche Kraft oder einen bereits zurückgelegten Verschiebeweg drahtlos zu übermitteln oder zu senden. Grundsätzlich bedeutet die Formulierung, dass der "Antrieb innerhalb des Rumpfes angeordnet" ist, dass sämtliche Teile des Antriebs, also beispielsweise Stator und Rotor einer elektrischen Maschine, innerhalb des Rumpfes angeordnet sind. Typischerweise ist der Antrieb vollständig in dem Rumpf aufgenommen. Bei weiteren Ausführungsformen ist der Antrieb nur teilweise innerhalb des Rumpfes aufgenommen. Typische Antriebe von weiteren Ausführungsformen umfassen einen magnetischen Antrieb, bei welchem der Antrieb innerhalb des Rumpfes Magnete aufweist, welche durch ein außerhalb des Marknagels und gegebenenfalls auch außerhalb des Körpers erzeugtes Magnetfeld bewegt oder betätigt werden. Bei weiteren Ausführungsformen ist als Antrieb eine Formgedächtnislegierung oder ein anderer Antrieb, beispielsweise ein piezoelektrischer Aktuator vorgesehen. Sowohl piezoelektrische Aktuatoren als auch elektrische Maschinen oder auch Formgedächtnislegierungen werden typischerweise über eine elektrische Verbindung mit Energie versorgt.

Bei typischen Ausführungsformen ist das erste Innenteil in einem mittleren Bereich des Rumpfes angeordnet. Dies ermöglicht eine Befestigung oder Fixierung eines Mittefragments an dem ersten Innenteil. Typischerweise ist das zweite Innenteil in einem zweiten Endbereich des Rumpfes angeordnet. So wird eine Fixierung oder Befestigung des zweiten Innenteils an einem zweiten Endfragment ermöglicht.

Typischerweise weisen das erste Innenteil und das zweite Innenteil eine Zylinderform auf. Bei üblichen Ausführungsformen ist der Durchmesser des ersten Innenteils und des zweiten Innenteils identisch. Bei bevorzugten Ausführungsformen weisen die Innenteile keine Ausformungen auf, die Innenteile sind also ausformungslos ausgebildet. Dies bietet den Vorteil einer einfachen Herstellung und eines sicheren Vermeidens von Verkantungen oder Ähnlichem. Es ist jedoch auch möglich, Nasen vorzusehen, um zusätzlich zu den Fixierungsbolzen die Lage der Innenteile in dem Rumpf vorzugeben. Der Ausdruck "Ausformungslos" bezieht sich dabei auf die äußere Begrenzungsfläche des jeweiligen Innenteils, insbesondere das erste Innenteil weist üblicherweise ein Innengewinde auf. Bei typischen Ausführungsformen weisen beide Innenteile einen Außendurchmesser auf, welcher im Wesentlichen dem Innendurchmesser des Rumpfes entspricht. Typischerweise ist der Außendurchmesser maximal 1 mm oder maximal 0,5 mm kleiner als der Innendurchmesser des hohlen Rumpfes.

Bei typischen Ausführungsformen ist zumindest eines der Innenteile, ggf. mit dem Antrieb, verdrehbar in dem Rumpf aufgenommen. Eine Führung wird ausschließlich durch die radial eingesetzten Fixierungsbolzen oder Fixierungsschrauben und durch die Langlöcher erreicht. Dies vereinfacht den Aufbau. Bei weiteren Ausführungsformen weist zumindest eines der Innenteile eine Führung auf, welche ein Verdrehen des Innenteils in dem Rumpf verhindert. Dies kann die Montage erleichtern.

Das erste Innenteil ist typischerweise als Hülse ausgebildet, welche über ein Innengewinde zum Eingriff mit einer Spindel verfügt. Typischerweise ist Abtriebsseitig des Getriebes eine Spindel vorgesehen. Die Spindel stellt typischerweise eine mechanische Verbindung zwischen dem Antrieb oder dem zweiten Innenteil einerseits und dem ersten Innenteil andererseits dar. Bei weiteren Ausführungsformen ist das zweite Innenteil mit einem Innengewinde für eine Spindel ausgerüstete, insbesondere bei Ausführungsformen, bei welchen der Antrieb mit dem ersten Innenteil integriert ausgeführt ist.

Typische Ausführungsformen umfassen als erstes Innenteil einen Riegel zum Fixieren des Knochensegmentes. Ein solcher Riegel bietet den Vorteil eines einfachen Aufbaus. Der Riegel ist vorzugsweise quer zur Richtung einer Spindel des Antriebs ausgerichtet. Der Riegel bildet somit vorzugsweise einstückig sowohl das erste Innenteil als auch das Fixierungsmittel, da er unmittelbar in einem Mittelfragment verankert wird. Beispielhafte Ausführungsformen mit mehrteiligem Riegel umfassen ein Innenteil, typischerweise ähnlich einer Mutter, und einen radial abstehendes Riegelelement. Hierzu ist der Riegel typischerweise mit einer Ausnehmung versehen. Diese Ausnehmung greift vorzugsweise formschlüssig in ein auf der Spindel sitzendes Innenteil, welches als Gewindeelement mit einem mittig angeordneten Innengewinde ausgeführt ist, ein. Diese Ausführungsform erlaubt eine Reduzierung der Baulänge oder die Möglichkeit, größere Distraktionsstrecken zu realisieren.

Üblicherweise weist zumindest eines der Innenteile, typischerweise beide Innenteile zumindest eine in radialer Richtung ausgerichtete Durchgangsöffnung zur Aufnahme eines Fixierungsbolzens auf. Die Durchgangsöffnungen sind bei Ausführungsformen Teil der Fixierungsmittel der Innenteile. Bei typischen Ausführungsformen weist zumindest eines Innenteil zwei oder mehr in radialer Richtung und parallel ausgerichtete Durchgangsöffnungen zur Aufnahme von Fixierungsbolzen auf. Dies bietet den Vorteil, dass das zweite Endfragment und das Mittelfragment sicher fixiert werden können. Bei weiteren Ausführungsformen weist zumindest eines der Innenteile genau eine radiale Durchgangsöffnung auf. Dies erleichtert bei einer Operation die Fixierung eines Fragments.

Bei typischen Ausführungsformen erfolgt eine Energiezuführung zu dem Antrieb mittels einer elektrischen Verbindung, welche innerhalb des Rumpfes geführt ist. Dies ermöglicht eine Energiezuführung ohne weitere Schädigung des Knochens. Typischerweise ist die elektrische Verbindung durch das zweite Innenteil hindurch geführt ist, beispielsweise durch eine Axialbohrung des zweiten Innenteils. Auf diese Weise wird die elektrische Verbindung vor Beschädigungen geschützt. Bei weiteren Ausführungsformen erfolgt eine Energiezuführung durch das erste Innenteil hindurch.

Vorzugsweise ist an einem Ende des Rumpfes eine Herzogkrümmung vorgesehen. Dies bietet die Möglichkeit, den Marknagel auch in einem Unterschenkelknochen (Tibia) einzusetzen. Vorzugsweise ist die Herzogkrümmung am Ende des Antriebs vorgesehen. Bei typischen Ausführungsformen ist der Antrieb gegenüberliegend zum zweiten Innenteil angeordnet, das heißt, dass das erste Innenteil zwischen dem zweiten Innenteil und dem Antrieb liegt. Vorzugsweise ist der Rumpf mit Ausnahme der Herzogkrümmung und der Öffnungen zur Durchführung von Bolzen oder Verriegelungsmitteln ideal zylindrisch. Dabei bedeutet "ideal zylindrisch", dass insbesondere keine sonstigen Stifte oder Nasen oder Längsöffnungen an dem Rumpf vorgesehen sind. Bei typischen Ausführungsformen ist der Marknagel derart ausgebildet, dass die Herzogkrümmung und der Antrieb proximal angeordnet sind. Typischerweise ist der Antrieb distal, d.h. hinter oder unterhalb, der Herzogkrümmung angeordnet.

### Kurze Beschreibung der Zeichnungen

Nachfolgend werden Ausführungsformen anhand der beiliegenden Figuren näher erläutert, wobei die Figuren zeigen:

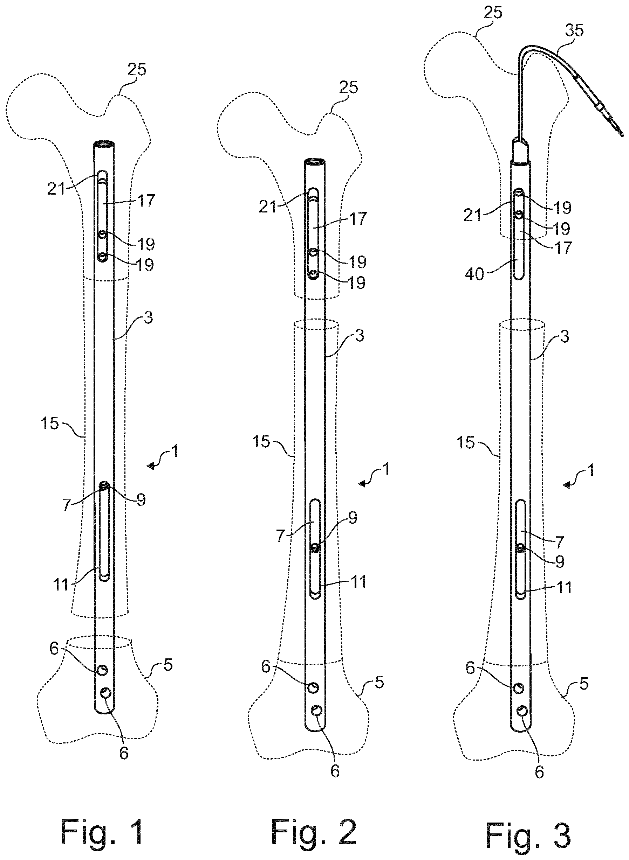

- Figuren 1 - 3: zeigen in schematischen Ansichten einen Marknagel in einer typischen Ausführungsform in drei unterschiedlichen Betriebszuständen.

### Beschreibung von Ausführungsbeispielen

Nachfolgend werden anhand der Figuren Ausführungsbeispiele erläutert, wobei für gleiche oder ähnliche Teile gleiche Bezugszeichen verwendet werden. Gleiche oder ähnliche Teile werden unter Umständen nicht im Zusammenhang mit jeder Figur erneut erläutert.

In den Figuren 1-3 ist eine typische Ausführungsform eines Marknagels 1 in drei verschiedenen Betriebszuständen gezeigt. Strichliert sind Fragmente eines Oberschenkelknochens gezeigt. Diese Fragmente sind nicht Teil des Ausführungsbeispiels und dienen lediglich der Illustration.

Der Marknagel 1 umfasst einen teilweise hohlen Rumpf 3. Der Rumpf 3 ist abgesehen von einem ersten Endbereich in axialer Richtung durchgehend hohl mit gleichbleibendem Innendurchmesser ausgebildet.

Weitere Ausführungsbeispiele von Marknägeln umfassen Rümpfe, welche innenliegende Stufen oder Absätze umfassen, beispielsweise um Endanschläge für Innenteile zu schaffen. Auf diese Weise können Verschiebungsbereiche der Innenteile begrenzt werden.

Der Rumpf 3 weist in einem ersten Endbereich des Rumpfes 3 Verriegelungsmittel auf, mit welchen der Rumpf 3 in einem ersten Endfragment 5 des Knochens verriegelbar ist. Die Verriegelungsmittel umfassen zwei radiale Bohrungen 6, welche in dem ersten Endbereich des Rumpfes 3 angeordnet sind. In die Bohrungen 6 werden bei einer Verwendung des Marknagels 1 Verriegelungsbolzen eingesetzt, welche eine fixierte Verbindung zwischen dem ersten Endfragment 5 und dem Rumpf 3 gewährleisten. In den Figuren 1-3 sind keine Verriegelungsbolzen gezeigt. Als Verriegelungsbolzen können aus dem Stand der Technik bekannte Bolzen zur Fixierung von Marknägeln in Knochen eingesetzt werden.

Grundsätzlich beziehen sich hierin die Begriffe "radial" und "axial" auf die Längsachse des Rumpfes des Marknagels.

In einem Mittelbereich des Rumpfes 3 ist ein erstes Innenteil 7 aufgenommen, von welchem in der Figur 1 lediglich ein kurzes Teilstück zu sehen ist. Das erste Innenteil 7 ist zylinderförmig mit einem Außendurchmesser, welcher lediglich geringfügig kleiner ist als der Innendurchmesser des in diesem Bereich hohlen Rumpfes 3. Das erste Innenteil 7 weist ein erstes Fixierungsmittel auf, welches eine radiale Durchgangsöffnung 9 umfasst, in welche ein Fixierungsbolzen eingesetzt werden kann.

Grundsätzlich sind für die Fixierungsbolzen von Ausführungsformen gleiche oder lediglich in den Abmessungen unterschiedliche Bolzen verwendbar, wie sie aus dem Stand der Technik zum Befestigen von Marknägeln in Knochen bekannt sind.

In dem mittleren Beriech des Rumpfes 3 weist der Rumpf 3 ein erstes Paar axial ausgerichteter, gegenüberliegender Langlöcher 11 auf. Das erste Paar Langlöcher 11 dient dazu, einen Durchtritt eines Fixierungsbolzens, welcher auch durch die Durchgangsöffnung 9 geschoben wird, aufzunehmen. Auf diese Weise ist es möglich, ein Mittelfragment 15 des Knochens fixiert mit dem ersten Innenteil 7 zu verbinden. Weiterhin wird auf diese Weise das erste Innenteil 7 am Verdrehen gehindert.

Eine Verdrehsicherung durch Fixierungsbolzen in den Langlöchern kann bei Ausführungsformen vorteilhaft sein, insbesondere falls eines der Innenteile ein Innengewinde aufweist, welches mit einer Spindel eines Antriebs zusammenwirkt. Auf diese Weise wird ein Rotations-Widerlager geschaffen.

Der Antrieb wird im Zusammenhang mit der Figur 3 erläutert, wohingegen die Spindel und das Innengewinde in den Figuren 1-3 nicht dargestellt sind, da sie innerhalb des Rumpfes 3 oder des ersten Innenteils 7 liegen.

In einem zweiten Endbereich ist ein zweites Innenteil 17 vorgesehen, welches weitere Durchgangsöffnungen 19 umfasst. Die zwei weiteren Durchgangsöffnungen 19 des zweiten Innenteils 17 dienen dazu, mit weiteren Fixierungsbolzen zusammen zu wirken, welche zum Fixieren eines zweiten Endfragments 25 des Knochens verwendet werden. In dem zweiten Endbereich des Rumpfes 3 ist ein zweites Paar Langlöcher 21 angeordnet, durch welche die weiteren Fixierungsbolzen hindurchgreifen können. Das zweite Innenteil 17 wird damit an einem Verdrehen gehindert. Auf diese Weise wird ein Wiederlager für den rotatorischen Antrieb geschaffen.

Aus dem zweiten Innenteil 17 ist eine elektrische Verbindung 35 in Form eines Kabels herausgeführt. Innerhalb des Innenteils 18 ist eine axiale Bohrung vorgesehen, welche außermittig liegt, so dass die axiale Bohrung außerhalb des Bereichs der mittig bezüglich der Längsachse ausgerichteten, radialen Durchgangsöffnungen 19 des zweiten Innenteils angeordnet ist.

Die elektrische Verbindung 35 dient dazu, den Antrieb 40 mit Strom zu versorgen und ihn außerdem zu kontrollieren. Weiterhin kann über die elektrische Verbindung 35 auch ein Sensorsignal geleitet werden, beispielsweise eines Kraftsensors, welcher eine Kraft zwischen dem ersten Innenteil 7 und dem zweiten Innenteil 17 in axialer Richtung erfasst.

Bei einer Verwendung des Marknagels 1 wird dieser wie in der Figur 1 schematisch gezeigt in einem Knochen angeordnet und mit Verriegelungsbolzen und Fixierungsbolzen mit den Knochenfragmenten, also den Endfragmenten 5 und 25 sowie dem Mittelfragment 15 verbunden. Im Betrieb treibt der Antrieb 40 die beiden Innenteile 7 und 17 auseinander, wobei die beiden Innenteile 7 und 17 mit dem Antrieb schwimmend, also frei verschieblich in axialer Richtung gelagert sind. Mit dem Antrieb 40 ist auch ein Zusammenziehen der Innenteile 7 und 17 möglich.

Das Mittelfragment 15 wird durch die sich auseinanderbewegenden Innenteile 7 und 17 in den Figuren 1-3 nach unten im Körper nach distal verschoben, bis das Mittelfragment 15 an dem ersten Endfragment 5 anstößt und dort sich abstützt. Währenddessen wird Knochen im Spalt zwischen dem Mittelfragment 15 und dem zweiten Endfragment 25 neu gebildet. Typische Geschwindigkeiten für die Relativbewegung der Innenteile 7 und 17 betragen zwischen 0,2 mm und 2,5 mm pro Tag, insbesondere zwischen 0,5 mm und 1,5 mm pro Tag.

Durch das Anstoßen oder Abstützen des Mittelfragments 15 an dem ersten Endfragment 5, siehe Figur 2, kann es zu einem Zusammenwachsen des Mittelfragmentes 15 mit dem ersten Endfragment 5 kommen, eventuell unterstützt durch eine Anpresswirkung oder Kompression. Während des Auseinandertreibens der Innenteile 7 und 17 ist in dem proximalen Spalt zwischen dem zweiten Endfragment 25 und dem Mittelfragment 15 bereits Knochen nachgewachsen.

Sobald das Mittelfragment 15 das erste Endfragment 5 erreicht hat, bewirkt das weitere Auseinanderbewegen der beiden Innenteile 7 und 17, dass das zweite Endfragment 25 nach proximal, in den Figuren 1-3 nach oben, getrieben wird. Dies ist schematisch in der Figur 3 dargestellt.

Mit Ausführungsformen erfindungsgemäßer Marknägel lassen sich auch bei kurzen Längen der Endfragmente große Knochendefekte mit einem Eingriff zum Einsetzen des Marknagels verschließen. Es sind keine Zwischen-Operationen notwendig, um Verlängerungen oder ähnliches durchzuführen.

In der Figur 3 ist das zweite Innenteil 17 derart weit nach proximal verschoben, dass der Antriebsteil mit dem Antrieb 40 des zweiten Innenteils in dem Bereich des zweiten Paares Langlöchern 21 gelangt. Der Antrieb 40 ist integral mit dem zweiten Innenteil 17 ausgeführt, das heißt das zweite Innenteil 17 umfasst den Antrieb 40. Die Spindel des Antriebs 40 verbindet das erste Innenteil 7 mit dem zweiten Innenteil 17 und treibt bei einer Rotation des Antriebs 40, welcher über ein Planetengetriebe zur Untersetzung verfügt, die beiden Innenteile 7 und 17 auseinander.

Die elektrische Verbindung 35 ist mit einem Empfänger verbunden, welcher subkutan angeordnet wird, um eine Kontrolle und eine Energieversorgung des Marknagels 1 über einen extrakorporalen Sender zu ermöglichen.

Die Erfindung ist nicht auf die zuvor beschriebenen Ausführungsbeispiele beschränkt, vielmehr wird der Umfang der Erfindung durch die Ansprüche bestimmt. Insbesondere sind nicht alle dargestellten Teile notwendigerweise Merkmale der Erfindung, dies gilt in besonderem Maße für die dargestellten menschlichen Knochen.

## Patentansprüche

1. Marknagel (1) für eine Distraktion mit Segmenttransport und anschließender Verlängerung eines Röhrenknochens, wobei der Marknagel (1) umfasst:
- einen sich in einer axialen Richtung des Marknagels (1) erstreckenden zumindest teilweise hohlen Rumpf (3),
- ein Verriegelungsmittel zum Verriegeln des Rumpfes (3) in einem ersten Endfragment (5) des Röhrenknochens,
- ein erstes Innenteil (7), welches innerhalb des Rumpfes (3) in axialer Richtung verschiebbar angeordnet ist und ein erstes Fixierungsmittel zum Fixieren eines Mittelfragments (15) des Röhrenknochens umfasst,
- ein zweites Innenteil (17), welches innerhalb des Rumpfes in axialer Richtung verschiebbar angeordnet ist und ein zweites Fixierungsmittel zum Fixieren eines zweiten Endfragments (25) des Röhrenknochens umfasst, und
- einen Antrieb (40) zum axialen Verschieben des ersten Innenteils (7) relativ zu dem zweiten Innenteil (17), **dadurch gekennzeichnet, dass** der Antrieb (40) entweder mit dem ersten Innenteil (7) oder mit dem zweiten Innenteil (17) integral ausgeführt ist,
- die Spindel des Antriebs (40) das erste Innenteil (7) mit dem zweiten Innenteil (17) verbindet, und
- der Antrieb (40) innerhalb des Rumpfes (3) zwischen dem ersten Fixierungsmittel des ersten Innenteils (7) und dem zweiten Fixierungsmittel des zweiten Innenteils (17) angeordnet ist.

2. Marknagel (1) nach Anspruch 1, wobei das Verriegelungsmittel in einem ersten Endbereich des Rumpfes (3) angeordnet ist.

3. Marknagel (1) nach einem der vorhergehenden Ansprüche, wobei das erste Innenteil (7) in einem mittleren Bereich des Rumpfes angeordnet ist und/oder wobei das zweite Innenteil (17) in einem zweiten Endbereich des Rumpfes angeordnet ist.

4. Marknagel (1) nach einem der vorhergehenden Ansprüche, wobei in dem mittleren Bereich des Rumpfes (3) ein erstes Paar axial ausgerichtete, gegenüberliegende Langlöcher (11) angeordnet sind und/oder wobei in dem zweiten Endbereich des Rumpfes (3) ein zweites Paar axial ausgerichtete, gegenüberliegende Langlöcher (21) angeordnet sind.

5. Marknagel (1) nach einem der vorhergehenden Ansprüche, wobei eine Energiezuführung zu dem Antrieb mittels einer elektrischen Verbindung (35) erfolgt, welche innerhalb des Rumpfes (3) geführt ist.

6. Marknagel (1) nach einem der vorhergehenden Ansprüche, wobei die elektrischen Verbindung (35) durch das zweite Innenteil (17) hindurch geführt ist.

7. Marknagel (1) nach einem der vorhergehenden Ansprüche, wobei das erste Fixierungsmittel des ersten Innenteils (7) zumindest eine in radialer Richtung ausgerichtete Durchgangsöffnung (9) zur Aufnahme eines Fixierungsbolzens umfasst.

8. Marknagel (1) nach einem der vorhergehenden Ansprüche, wobei das zweite Fixierungsmittel des zweiten Innenteils (17) zumindest eine, insbesondere mindestens zwei, in radialer Richtung ausgerichtete Durchgangsöffnungen (19) zur Aufnahme jeweils eines Fixierungsbolzens umfasst.

9. Marknagel (1) nach einem der vorhergehenden Ansprüche, wobei das erste Innenteil (7) und/oder das zweite Innenteil (17) jeweils eine Zylinderform aufweisen

10. Marknagel (1) nach einem der vorhergehenden Ansprüche, wobei das Verriegelungsmittel Bohrungen (6) und/oder Verriegelungsbolzen umfasst, welche ausschließlich radial ausgerichtet sind.

## Claims

1. Medullary pin (1) for a distraction with segment transport and subsequent lengthening of a tubular bone, wherein the medullary pin (1) comprises:
- an at least partly hollow body (3) extending in an axial direction of the medullary pin (1),
- a locking means for locking the body (3) in a first end fragment (5) of the tubular bone,
- a first inner part (7), which is arranged displaceably in the axial direction inside the body (3) and comprises a first fixation means for the fixation of a middle fragment (15) of the tubular bone,
- a second inner part (17), which is arranged displaceably in the axial direction inside the body and comprises a second fixation means for the fixation of a second end fragment (25) of the tubular bone, and
- a drive unit (40) for the axial displacement of the first inner part (7) relative to the second inner part (17),
**characterized in that**
- the drive unit (40) is integrated either with the second inner part (17) or with the first inner part (7),
- the spindle of the drive unit (40) connects the first inner part (7) with the second inner part (17), and
- the drive unit (40) is arranged inside the body (3) between the first fixation means of the first inner part (7) and the second fixation means of the second inner part (17).

2. Medullary pin (1) according to claim 1, wherein the locking means is arranged in a first end region of the body (3).

3. Medullary pin (1) according to one of the preceding claims, wherein the first inner part (7) is arranged in a middle region of the body and/or the second inner part (17) is arranged in a second end region of the body.

4. Medullary pin (1) according to one of the preceding claims, wherein a first pair of axially extending oblong holes (11) opposite each other is arranged in the middle region of the body (3) and/or a second pair of axially extending oblong holes (21) opposite each other is arranged in the second end region of the body (3).

5. Medullary pin (1) according to one of the preceding claims, wherein a supply of energy to the drive unit occurs by way of an electrical connection (35), which is arranged inside the body (3).

6. Medullary pin (1) according to one of the preceding claims, wherein the electrical connection (35) is led through the second inner part (17).

7. Medullary pin (1) according to one of the preceding claims, wherein the first fixation means of the first inner part (7) has at least one through opening (9) extending in the radial direction to accommodate a fixation bolt.

8. Medullary pin (1) according to one of the preceding claims, wherein the second fixation means of the second inner part (17) has at least one, preferably at least two through openings (19) oriented in the radial direction to accommodate each fixation bolt.

9. Medullary pin (1) according to one of the preceding claims, wherein the first inner part (7) and/or the second inner part (17) have a cylindrical shape.

10. Medullary pin (1) according to one of the preceding claims, wherein the locking means comprises boreholes (6) and/or locking bolts which are oriented exclusively radially.

## Revendications

1. Clou médullaire (1) pour une distraction avec transport de segment et allongement successif d'un os long, le clou médullaire (1) comportant:
- un tronc au moins partiellement creux (3) s'étendant dans une direction axiale du clou médullaire (1),
- un moyen de verrouillage destiné à verrouiller le tronc (3) dans un premier fragment d'extrémité (5) de l'os long,
- une première partie intérieure (7) qui est disposée de manière déplaçable dans la direction axiale à l'intérieur du tronc (3) et qui comporte un premier moyen de fixation destiné à fixer un fragment central (15) de l'os long,
- une deuxième partie intérieure (17) qui est disposée de manière déplaçable dans la direction axiale dans le tronc et un deuxième moyen de fixation destiné à fixer un deuxième fragment d'extrémité (25) de l'os long, et
- un entraînement (40) destiné à déplacer axialement la première partie intérieure (7) par rapport à la deuxième partie intérieure (17),
**caractérisé par le fait que**
- l'entraînement (40) est réalisé solidaire soit de la première partie intérieure (7), soit de la deuxième partie intérieure (17),
- la broche de l'entraînement (40) connecte la première partie intérieure (7) à la deuxième partie intérieure (17), et
- l'entraînement (40) est disposé à l'intérieur du tronc (3) entre le premier moyen de fixation de la première partie intérieure (7) et le deuxième moyen de fixation de la deuxième partie intérieure (17).

2. Clou médullaire (1) selon la revendication 1, dans lequel le moyen de verrouillage est disposé dans une première partie d'extrémité du tronc (3).

3. Clou médullaire (1) selon l'une des revendications précédentes, dans lequel la première partie intérieure (7) est disposée dans une zone centrale du tronc et/ou dans lequel la deuxième partie intérieure (17) est disposée dans une deuxième zone d'extrémité du tronc.

4. Clou médullaire (1) selon l'une des revendications précédentes, dans lequel sont disposés, dans la partie centrale du tronc (3), une première paire de trous oblongs opposés alignés axialement (11) et/ou dans lequel sont disposés, dans la deuxième zone d'extrémité du tronc (3), une deuxième paire de trous oblongs opposés axialement alignés (21).

5. Clou médullaire (1) selon l'une des revendications précédentes, dans lequel une alimentation d'énergie vers l'entraînement a lieu au moyen d'une connexion électrique (35) qui est guidée à l'intérieur du tronc (3).

6. Clou médullaire (1) selon l'une des revendications précédentes, dans lequel la connexion électrique (35) est guidée à travers la deuxième partie intérieure (17).

7. Clou médullaire (1) selon l'une des revendications précédentes, dans lequel le premier moyen de fixation de la première partie intérieure (7) comporte au moins une ouverture de passage (9) orientée dans la direction radiale destinée à recevoir un boulon de fixation.

8. Clou médullaire (1) selon l'une des revendications précédentes, dans lequel le deuxième moyen de fixation de la deuxième partie intérieure (17) comporte au moins une, en particulier au moins deux, ouvertures de passage (19) orientées dans la direction radiale destinées à recevoir, chacune, un boulon de fixation.

9. Clou médullaire (1) selon l'une des revendications précédentes, dans lequel la première partie intérieure (7) et/ou la deuxième partie intérieure (17) présentent, chacune, une forme cylindrique.

10. Clou médullaire (1) selon l'une des revendications précédentes, dans lequel le moyen de verrouillage comporte des alésages (6) et/ou des boulons de verrouillage qui sont exclusivement orientés radialement.
